# EUROPEAN PATENT APPLICATION

(11) **EP 1 175 879 A1**
(43) Date of publication of application: **30.01.2002**
(21) Application number: 01904670.5
(22) Date of filing: 01.02.2001
(51) Int. Cl.: A61F 9/008

(54) **KOURENKOV'S CANNULA USED FOR A METHOD FOR AN OPERATION OF REFRACTORY-CORRELATING EXIMER-LASER INTRASTROMAL KERATECTOMY (REIK)**

(30) Priority: 02.02.2000 RU 2000102334; 31.01.2001 RU 2001102703
(71) Applicant: Ooo Meditsinsky Nauchno-Issledovatelsky Oftalmolog Ichesky Tsentr "Novy Vzglyad", Moscow, 121614 (RU)
(72) Inventor: KURENKOV, Vyacheslav Vladimirovich, Moscow, 119121 (RU)
(74) Representative: Burford, Anthony Frederick
(86) International application number: RU0100038
(87) International publication number: WO0156520

(57) **Abstract**

The invention relates to medical instruments and can be used for an operation of refractory-correlating eximer-laser intrastromal keratectomy (REIK). The inventive cannula comprises a hollow body, a curved working part, side channels and a cavity connected to the hollow body. The side channels are used for instillating and/or irrigating the cornea flap while performing the refractory-correlating eximer-Iaser intrastromal keratectomy (REIK). Longitudinal axes of the side channels are placed across a plane of the working part bending and oriented angle-wise or angles-wise with respect to the longitudinal axis of the working part. The working part has a tapered end for facilitating its introduction and/or a separation of tissues. The body and/or the working part are embodied with equal cross-sections and/or with cross-sections whose shapes differ from each other.

## Description

The present invention relates to the field of ophthalmology and makes it possible to perform correction of vision with the aid of an excimer laser.

The invention relates to medical instruments and may be used in all fields of surgery, particularly during the operation of refraction-correcting intrastromal keratectomy by excimer laser (REIK) and other versions of intrastromal operations on the cornea.

Cannulas are intended for the conduct of diagnostic and therapeutic manipulations in natural shallow cavities of the body and fistulous passages. They are chiefly produced from metal much like needles and are provided with a trocar (mandrin).

In ophthalmology, cylindrical or impressed cannulas are used for irrigation of the lachrymal duct, suction of lenticular mass, and introduction of air and irrigation fluid into the anterior chamber of the eye.

Already known from the state-of-the-art is an aspirating needle incorporating a cannula, auxiliary tube, and radially bent working part, with several apertures being made on the convex surface of the working part and with the end section of the working part being directed towards the convex surface. The aspirating needle incorporates a limiter between the auxiliary tube and working part (see Russian Federation Patent No. 543395 cl. A 61 B 17/34 published on 25.01.1977).

The nearest technical solution to the invention applied for is a cannula for conducting the operation of intrastromal keratomylesis by laser (LASIK). The cannula, used to irrigate the cornea of the eye, incorporates an irrigation solution source, a tube which is manually transferred and has sufficient length for penetration into the subgraft region in the upper part of the cornea of the eye that is formed by surgical intervention. The end of the tube represents a flattened head, at the top of which apertures are made to admit irrigation fluid as well as two apertures arranged above and below to admit fluid upwards and downwards. The aforesaid upper and lower apertures are arranged at an angle of 90° from the axis passing through the aperture on top of the head and on a single straight line. In another version, they may be displaced upwards or downwards (US Patent No. 5755700, cl. 604-257, published on 26.05.1998).

A deficiency of the known cannula is non-uniformity of delivery and distribution of the irrigation fluid under the graft, which entails retention under the graft of particles of tissue that have been subjected to the action of the excimer laser together with organic particles of conjunctival content.

The technical result of the proposed cannula design is uniform distribution of irrigation fluid under the graft and achievement of full cleansing of the subgraft space as well as creation of a flow of irrigation fluid in one direction, thus causing a reduction in the possibility of mixing with conjunctival fluid and infiltration into the latter of organic particles.

The aforesaid result is achieved in Kurenkov's cannula -- a cannula, incorporating a hollow body and bent working part having lateral ducts and a cavity communicating with the body cavity, with the lateral ducts being arranged for instillation and/or irrigation of the subgraft space during conduct of refraction-correcting intrastromal keratectomy by excimer laser (REIK), including the conduct of instillation anaesthesia, cleansing of the conjunctival cavity, installation of a vacuum ring, execution of a section with formation of a graft on the limb, opening of the graft, conduct of laser keratoablation, instillation of medications and repositioning of the graft, with the anaesthesia being conducted with the aid of a vasoconstrictor, the vacuum ring being arranged so that its longitudinal axis passes through the centre of the pupil, after which the ring is displaced downwards along the vertical by 0.5-1.0 mm, before formation of the graft, a mixture of solutions of 0.4-0.6% methylcellulose and 0.17-0.19% sodium hyaluronate is instilled in the conjunctival cavity, with foundation of the graft being arranged at 12 o'clock and the lower margin of the graft being required to have a distance from the limbus not greater than 2 mm, before the graft is opened, it is folded in half, the inside surface inwards, after laser keratoablation, the stromal bed is irrigated with agents improving regeneration, and, after repositioning, a solution of an antibiotic and non-steroid medications is instilled, the graft is then transpalpebrally smoothed by hand, with the axes of the lateral ducts of the cannula being arranged across the bending plane of its working part and being oriented longitudinally at an angle or angles to the longitudinal axis of the working part, with the working part having a tapering end for introduction and/or separation of tissues, and the body and/or working part being arranged with identical cross-sections and/or with cross-sections whose shapes differ from each other.

The cannula has a working part bend of 130°-150°.

The cannula has longitudinal axes of lateral ducts oriented to the longitudinal axis of the working part at 30°-50°.

The lateral ducts of the cannula have a shaped cross-section or shaped cross-sections in the form of circles and/or ovals and/or polygons.

The cannula is made of metal.

The cannula is used in the operation of refraction-correcting intrastromal keratectomy by excimer laser (REIK).

Fig. 1 shows a general view of Kurenkov's cannula.

Fig. 2 shows Kurenkov's cannula in which the apertures near the end are arranged at an angle of 90°.

Fig. 3 shows Kurenkov's cannula in which the working part is arranged with a radial bend.

Fig. 4 shows an arrangement of Kurenkov's cannula for irrigation of the subgraft space.

Kurenkov's cannula incorporates hollow body 1 and bent working part 2. The working part has lateral ducts 3 and cavity 4, which communicates with cavity 5 of body 1. The axes of the lateral ducts of the cannula are arranged across the bending plane of its working part and oriented longitudinally at an angle or angles to the longitudinal axis of working part 2. The working part has a tapering end for introduction and/or separation of tissues. Body 1 and/or working part 2 are arranged with identical cross-sections and/or with cross-sections whose shapes differ from each other. As indicated, the cannula is used during refraction-correcting intrastromal keratectomy by excimer laser (REIK) and other versions of intrastromal operations on the cornea.

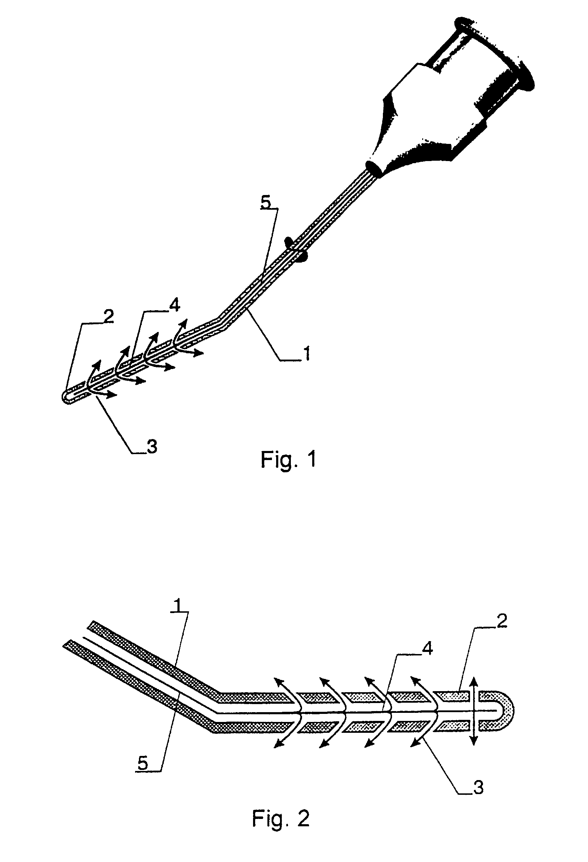

Kurenkov's cannula shown in Fig. 2 has apertures near the end of the cannula arranged at an angle of 90°. This cannula arrangement may usually be used for small sizes of graft.

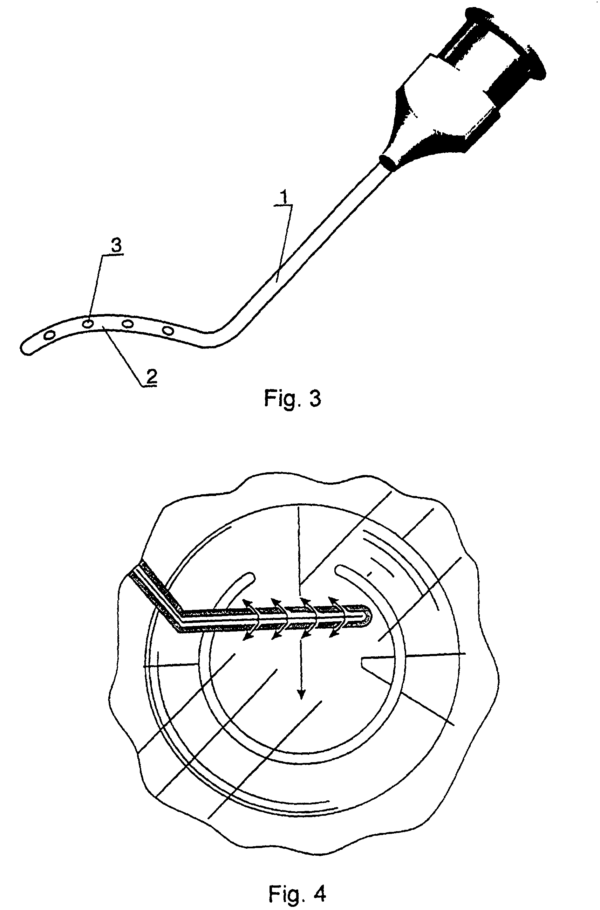

The cannula arrangement whose working part has a radial bend (Fig. 3) enables the subgraft space to be irrigated in the most painless way possible.

The cannula is used during the operation of refraction-correcting intrastromal keratectomy by excimer laser (REIK).

For refraction surgery, ophthalmology currently uses lasers operating on an argon-fluorine mixture with a long emission wavelength of 193 nm. Most promising among the latter is the Nidek - 5000 2c laser. Use of low-density energy should be included among its attributes. This makes it possible to reduce corneal trauma and reduce acoustic shock. Moreover, the temperature in the ablation zone scarcely rises at all. The beam delivery system excludes the formation of a central islet, which is often a complication during use of wide-profile lasers.

The method applied for is characterised by the following stages.

In the first stage, to obtain the most predicted refraction result and to reduce its error, it is necessary to modify the conditions of corneal anaesthesia and thereby exclude or significantly reduce the harmful and toxic effects of the anaesthetic on the epithelia as well as to reduce its diffusion into the stroma. Reduction of diffusion leads to a reduction in the effect of the anaesthetic on tissue hydration. Modification of constant hydration conditions affects the accuracy of excimer laser operation, since irrigation of the corneal tissue prevents programmed removal of tissue during one laser beam scan. However, the set of enumerated conditions impairs the quality of the microkeratome section because of the less dense consistency of the stroma, increasing the unevenness of the section surface. The toxic quality of the anaesthetic leads to maceration of the epithelium, which affects forthcoming adaptation of the graft through degradation of the sliding properties of the corneal surface.

To avoid or significantly reduce the aforesaid adverse conditions, it is proposed to reduce the quantity of anaesthetic instillations through application of a vasoconstrictor and consequently the time until the start of laser action as well, and thereby to prevent the onset of elevated hydration during the operation. In turn, the duration of action of the anaesthetic in droplet form in the complex with the vasoconstrictor should not exceed 2-3 minutes before the start of the procedure, which is due to the normal permeability time of the cornea. As distinct from the known method, the anaesthetic in the proposed invention is instilled for 2-3 minutes before the start of laser beam action and the operation time thereby curtailed. Application of a vasoconstrictor moreover enables the stromal bed drying stage to be avoided.

In the second stage, irrigation and cleansing of the conjunctival cavity are performed, with mechanical removal of foreign inclusions and detritus. Cleansing of the conjunctival surface is performed by irrigation with a balanced isotonic solution in a mixture together with an antibiotic through a dispersion cannula by a pressurised syringe, which reduces the risk of infection of the subgraft space after the operation. In this case, it is proposed to use not simply an isotonic solution, as proposed in the known method, but a mixture of isotonic solution and antibiotic solution.

The third stage relates to installation of the vacuum ring. The vacuum ring is installed on the cornea corresponding to the centre of the pupil and not the centre of the cornea, with downward displacement along the vertical. An applanation tonometer is used to confirm attainment of the optimum pressure in the eye. The lower margin of the corneal graft should have a distance from the limbus not greater than 2 mm, since, during the physiological blinking process and with regard for the standard position of the lower margin of the eyelid, the risk of the graft being displaced after its repositioning is reduced. In the later postoperative period, better fixing of the corneal graft to the stroma is found with such a vacuum ring arrangement.

The fourth stage of the operation concerns an intrastromal section with a lubricant. The section is only performed by the method using a lubricant, i.e. a substance with a lubricating action representing a mixture of a 0.4-0.6% methylcellulose solution and a 0.17-0.19% sodium hyaluronate solution. The selected concentrations enable the maximum effect to be produced during sliding of the graft. At lower concentrations, the solution will be liquid. At higher concentrations, it will be viscous and will not give any sliding effect. When the aforesaid lubricant is used, friction during excision of the corneal graft is significantly reduced, and damage to the epithelial surface of the graft is consequently also reduced.

In the fifth stage, the graft is separated and lifted by a method excluding any contact of the internal surface of the graft with the air. To achieve this, a spatula is introduced under the base of the graft at 12 o'clock, and the graft is displaced in the direction of 12 o'clock by being moved parallel and upwards towards the limbus in the corneal plane. The internal surface of the graft then does not make contact with the air. The graft is laid out at 12 o'clock and then folded in half, the inside surface inwards. As the graft slides over the intrastromal surface created, uniform distribution of fluid in an extremely thin layer occurs over the whole area, with the amount of moisture present in the section being optimum for creation on the surface of the stromal bed of a liquid layer acting as a natural mask smoothing the surface of the section. The surface thus created and smoothed by liquid is optimum for conduct of keratoablation.

In the proposed method, after the graft is lifted, there is no stage of surface drying by any method used in the known operation (scalpel, sponge, microsponge). There must be absolutely no contact with the surface, since this changes its smooth surface, which may lead to impairment of keratoablation quality.

The sixth stage of the operation concerns specialised photokeratoablation by excimer laser. In the proposed operation, it is only possible to use an excimer laser with a slit rotation-scanning system of beam delivery. A specific beam frequency and scanning area together with specific nomograms (computer programs for laser operation) are used.

This type of laser prevents liquid starting to boil on the surface and prevents excess moisture from collecting in the central zone and a central islet from forming. The speed of motion, angle of rotation, and pulse frequency of the laser beam exclude excessive heating of the surface and accordingly burning of tissue as well as favouring a smoother and more even surface as distinct from the known method.

The seventh stage of the operation concerns processing of the ablated surface.

Processing includes wetting and two stages of mechanical cleansing. After completion of ablation, the stromal surface is wetted with a balanced solution of medication improving the cornea regeneration process. The solution particularly used is carnosine, whose pH corresponds to that of the corneal tissue. This is followed by mechanical removal of any retained tissue particles present on the ablated surface with a corneal scraper. Movement should only ever be performed in the vertical direction from 12 o'clock to 6 o'clock without any return in the opposite direction. If the corneal scraper is moved up and down or at any angle in relation to the graft limb, the liquid on the surface of the stroma will form microripples, which will adversely affect refraction results after the operation.

Mechanical removal of microparticles should be concluded by the surface of the stromal section being irrigated with a solution of antioxidants, such as e.g. carnosine, by means of a dispersion cannula by a pressurised syringe. A layer of liquid is then formed on the surface of the cornea.

The eighth stage relates to repositioning of the graft by the airless contact method. The folded graft at 12 o'clock is straightened through being displaced uniformly downwards over its whole surface. The internal surfaces initially slide in relation to each other. The free margin then shifts to 6 o'clock. Through the graft sliding over the stromal surface, the liquid layer rises onto the surface of the corneal graft, excluding any air or microparticles from the ambient air from finding their way under the graft, optimum repositioning of the graft in the limb is thereby achieved. This method minimises the possibility of any unwanted inclusions from finding their way under the graft, thereby facilitating and curtailing subsequent irrigation of the subgraft space and consequently minimising the trauma factor.

The ninth stage refers to irrigation of the subgraft space. The cannula is introduced under the graft base at 12 o'clock. The corneal part of the cannula is arranged parallel to the graft limb. The end of the cannula should not extend as far as the graft margin and moreover cause it to lift. When the solution is delivered, movement of the liquid occurs in one direction towards the raised slit channel formed around the cannula body.

The tenth stage concerns instillation of medications.

Immediately after completion of the procedure, it is recommended to instill an antibacterial preparation together with a non-steroid anti-inflammatory and to remove the blepharostat immediately. This is to exclude exchange diffusion between the medication and capillary layer of the subgraft fluid, also with regard for possible resinification processes This makes it possible to retain a homogeneous thin layer, which directly influences uniform adaptation of the graft and the greatest acuity of visual functions. On the other hand, an inhomogeneous layer prevents optimum adaptation for a number of reasons, such as e.g. relative destruction of the spherical surface because of the disparity of media in the general plane of the layer after fixing of the given state blocks microexcursion by the process of epithelisation of the vapour of the graft groove, and the different time of disappearance (dissipation) of the capillary layer under the graft is accordingly a cause of loss of lines under normal refraction.

The eleventh stage concerns transpalpebral adaptation of the graft.

After removal of the blepharostat, the upper and lower eyelids are held in the fingers. Then, with the finger being pressed on the upper eyelid and the lower one being retained in place, a coercive movement is performed over the graft by the upper eyelid from 12 o'clock to 6 o'clock. On completion of the manipulation, it is released, with the eyelid returning into place of its own accord. The patient is then asked to blink while the lower eyelid is being retained in place. The lower eyelid is then released, with the eye continuing to be observed for 1-2 minutes. By this method, through ensuring uniform distribution of pressure over the whole area of the graft and through excess fluid being squeezed out from underneath, the most uniform distribution of residual subgraft fluid can be achieved. The proposed cannula may also be used for its introduction into tissue. As distinct from other known arrangements, the application of instruments and sponges is excluded, thereby avoiding the effects of harmful factors and minimising the possibility of incorrect adaptation.

### Example.

Patient K approached the clinic, complaining of deterioration of vision. On examination, the patient was found to have -5.0 D myopia in the left eye. The patient was offered the operation of refraction-correcting intrastromal keratectomy by excimer laser, to which he agreed. After the patient had been laid on the operation table, 0.5% proparacaine solution was instilled. After exposure for 10-20 seconds until the sensation of pinching had disappeared, a blepharostat was fitted. A secondary instillation of 1% proparacaine solution was performed when the eye was positioned to the fullest extent upwards, downwards, and directly forwards. 2 drops of adrenaline hydrochloride were then instilled at 1:1000 dilution. After exposure for 60 seconds, the conjunctival cavity was cleansed with an isotonic solution together with cipromed solution for 10-15 seconds. A vacuum ring was then immediately installed corresponding to the centre of the pupil with downward displacement along the vertical and also with regard for the fact that the lower margin of the graft was positioned 1.5 mm away from the limbus. The vacuum system was switched on. An applanation tonometer is used to confirm the intraocular pressure. Several drops of a mixture of 0.5% methylcellulose solution and 0.18% sodium hyaluronate solution were instilled. The previously assembled microkeratome head and motor were then fitted on the pin of the vacuum ring. The graft was then formed. The vacuum system was unsealed, and the assembled system was removed. The patient's eye should then have remained coaxially with the laser beam system. This was achieved by fixing the patient's gaze on a laser target to be aimed at. After removal of the system, the state of the cornea was visually assessed, and a spatula was introduced under the graft at the base or limb at 12 o'clock. Through the spatula being moved upwards in the plane of the cornea towards 12 o'clock, the graft was displaced and arranged so that it was folded in half with the inside surface inwards. The target of the excimer laser was set and centred with the visual axis. Ablation was then immediately initiated according to the nomogram produced for this patient and entered in the laser computer before the operation. After completion of ablation, the surface of the cornea was wetted with carnosine solution. A spatula was used for mechanical removal of microparticles retained on the ablated surface by a 12 o'clock to 6 o'clock movement without returning in the opposite direction. The surface was then irrigated with the aid of a dispersion cannula, and a liquid layer was created. The spatula was then applied to the graft bend and moved downwards to 6 o'clock so that the lower margin of the graft slid over the stromal surface and the liquid remained on the epithelial surface. After this manipulation was completed, the graft was completely straightened. In the following stage, the cannula was introduced under the base of graft in such a way as to form a slit aperture at the cannula introduction site while the opposite side remained connected with the stroma. The solution was supplied so that the cannula moved predominantly towards the slit aperture with the opposite side serving as an indicator of the pressure of the liquid circulating under the graft. It was opened only when the pressure became excessive and served as a signal of its reduction. The cannula was moved from 12 o'clock to 6 o'clock. The irrigation procedure was checked with a microscope. A solution of cipromide and diklof was instilled, after which the blepharostat was removed. The eyelids were manually fixed open. The lower eyelid remained fixed, while the upper eyelid was slightly pressed and closed. It was then released, returning of its own accord. With the patient's lower eyelid being held in position, the patient was asked to blink. The position of the graft was visually assessed. The patient was then taken off the table. The patient continued to be observed for two hours. The patient was kept under observation for a year, and no refraction changes were found.

Kurenkov's cannula proposed in this invention makes it possible to cleanse the subgraft space more effectively, since the subgraft space as a whole is irrigated. The flow of liquid is organised in one direction and contains no zone where particles of tissue present after action of the excimer laser can be retained.

## Claims

1. Cannula, incorporating a hollow body and bent working part having lateral ducts and a cavity communicating with the body cavity, **characterised in that** the lateral ducts are arranged for instillation and/or irrigation of the subgraft space during conduct of refraction-correcting intrastromal keratectomy by excimer laser (REIK), including the conduct of instillation anaesthesia, cleansing of the conjunctival cavity, installation of a vacuum ring, execution of a section with formation of a graft on the limb, opening of the graft, conduct of laser keratoablation, instillation of medications and repositioning of the graft, with the anaesthesia being conducted with the aid of a vasoconstrictor, the vacuum ring being arranged so that its longitudinal axis passes through the centre of the pupil, after which the ring is displaced downwards along the vertical by 0.5-1.0 mm, before formation of the graft, a mixture of solutions of 0.4-0.6% methylcellulose and 0.17-0.19% sodium hyaluronate is instilled in the conjunctival cavity, with foundation of the graft being arranged at 12 o'clock and the lower margin of the graft being required to have a distance from the limbus not greater than 2 mm, before the graft is opened, it is folded in half, the inside surface inwards, after laser keratoablation, the stromal bed is irrigated with agents improving regeneration, and, after repositioning, a solution of an antibiotic and non-steroid medications is instilled, the graft is then transpalpebrally smoothed by hand, with the axes of the lateral ducts of the cannula being arranged across the bending plane of its working part and being oriented longitudinally at an angle or angles to the longitudinal axis of the working part, with the working part having a tapering end for introduction and/or separation of tissues, and the body and/or working part being arranged with identical cross-sections and/or with cross-sections whose shapes differ from each other.
